# FASCICULE DE BREVET EUROPEEN

(11) **EP 2 638 164 B1**
(45) Date de publication et mention de la délivrance du brevet: **25.04.2018**
(21) Numéro de dépôt: 11799129.9
(22) Date de dépôt: 10.11.2011
(51) Int. Cl.: C12N 15/115, C12Q 1/68

(54) **LIGAND SPECIFIQUE DE L'ANNEXINE 2**
SPEZIFISCHER LIGAND FÜR ANNEXIN 2
SPECIFIC LIGAND FOR ANNEXIN 2

(30) Priorité: 12.11.2010 FR 1004412
(43) Date de publication de la demande: 18.09.2013
(73) Titulaire: Commissariat à l'Énergie Atomique et aux Énergies Alternatives, 75015 Paris (FR)
(72) Inventeur: DUCONGE, Frédéric, F-92330 Sceaux (FR); CIBIEL, Agnès, F-91440 Bures Sur Yvette (FR)
(74) Mandataire: Cabinet Plasseraud
(86) Numéro de dépôt international: PCT/IB2011/055019
(87) Numéro de publication internationale: WO 2012/063219

(56) Documents cités:
- WO-A1-2005/093097
- SHARMA ET AL: "Angiogenesis-associated protein annexin II in breast cancer: Selective expression in invasive breast cancer and contribution to tumor invasion and progression", EXPERIMENTAL AND MOLECULAR PATHOLOGY, vol. 81, no. 2, 1 octobre 2006 (2006-10-01), pages 146-156, XP005661084, ISSN: 0014-4800
- TAVITIAN BERTRAND ET AL: "In Vivo Imaging of Oligonucleotidic Aptamers", METHODS IN MOLECULAR BIOLOGY, HUMANA PRESS INC, NJ, US, vol. 535, 2009, pages 241-259, XP008138323, ISSN: 1064-3745
- LI CHAN ET AL: "A Cy5-Labeled S100A10 Tracer Used to Identify Inhibitors of the Protein Interaction With Annexin A2", ASSAY AND DRUG DEVELOPMENT TECHNOLOGIES, vol. 8, no. 1, février 2010 (2010-02), pages 85-95, XP002645214,
- PESTOURIE ET AL: "Aptamers against extracellular targets for in vivo applications", BIOCHIMIE, vol. 87, no. 9-10, 1 septembre 2005 (2005-09-01), pages 921-930, XP005074124, ISSN: 0300-9084
- KESAVAN KAMALA ET AL: "Annexin A2 Is a Molecular Target for TM601, a Peptide with Tumor-targeting and Anti-angiogenic Effects", JOURNAL OF BIOLOGICAL CHEMISTRY, vol. 285, no. 7, février 2010 (2010-02), pages 4366-4374, XP002645215, ISSN: 0021-9258 cité dans la demande
- Cibiel, A. et al.: "Fusion of cell-SELEX and in vivo imaging-based screening to identify both tumor-targeting agents and biomarkers", , 8 September 2011 (2011-09-08), Retrieved from the Internet: URL:http://www.wmis.org/abstracts/2011/dat a/papers/P149.html

### LISTAGE DES SEQUENCES

<110> Commissariat à l'Energie Atomique et aux Energies Alternatives
<120> LIGAND SPECIFIQUE DE L'ANNEXINE 2
<130> F263-408
<160> 11
<170> PatentIn version 3.5
<210> 1
   <211> 48
   <212> RNA
   <213> Séquence artificielle
<220>
   <223> Séquence centrale de l'aptamère ACE8
<400> 1
   ggaacgcaag aacugaggcc augaggcgcc uucccuugcu caggacgc 48
<210> 2
   <211> 49
   <212> RNA
   <213> Séquence artificielle
<220>
   <223> Séquence centrale de l'aptamère ER4
<400> 2
   agcuaggccg caaggugccu caacgccauc ugagugccga cccgaucgc 49
<210> 3
   <211> 95
   <212> RNA
   <213> Séquence artificielle
<220>
   <223> Séquence de l'aptamère ACE8
<400> 3
   gggagaugau ccguugaugc gagggaacgc aagaacugag gccaugaggc gccuucccuu 60
   gcucaggacg caagucgucg uucguaggca gaauc 95
<210> 4
   <211> 96
   <212> RNA
   <213> Séquence artificielle
<220>
   <223> Séquence de l'aptamère ER4
<400> 4
   gggagaugau ccguugaugc gagagcuagg ccgcaaggug ccucaacgcc aucugagugc 60
   cgacccgauc gcaagucguc guucguaggc agaauc 96
<210> 5
   <211> 339
   <212> PRT
   <213> Homo sapiens
<400> 5
   Met Ser Thr Val His Glu Ile Leu Cys Lys Leu Ser Leu Glu Gly Asp
   1               5                   10                  15
   His Ser Thr Pro Pro Ser Ala Tyr Gly Ser Val Lys Ala Tyr Thr Asn
               20                  25                  30
   Phe Asp Ala Glu Arg Asp Ala Leu Asn Ile Glu Thr Ala Ile Lys Thr
           35                  40                  45
   Lys Gly Val Asp Glu Val Thr Ile Val Asn Ile Leu Thr Asn Arg Ser
       50                  55                  60
   Asn Ala Gln Arg Gln Asp Ile Ala Phe Ala Tyr Gln Arg Arg Thr Lys
   65                  70                  75                  80
   Lys Glu Leu Ala Ser Ala Leu Lys Ser Ala Leu Ser Gly His Leu Glu
                   85                  90                  95
   Thr Val Ile Leu Gly Leu Leu Lys Thr Pro Ala Gln Tyr Asp Ala Ser
               100                 105                 110
   Glu Leu Lys Ala Ser Met Lys Gly Leu Gly Thr Asp Glu Asp Ser Leu
           115                 120                 125
   Ile Glu Ile Ile Cys Ser Arg Thr Asn Gln Glu Leu Gln Glu Ile Asn
       130                 135                 140
   Arg Val Tyr Lys Glu Met Tyr Lys Thr Asp Leu Glu Lys Asp Ile Ile
   145                 150                 155                 160
   Ser Asp Thr Ser Gly Asp Phe Arg Lys Leu Met Val Ala Leu Ala Lys
                   165                 170                 175
   Gly Arg Arg Ala Glu Asp Gly Ser Val Ile Asp Tyr Glu Leu Ile Asp
               180                 185                 190
   Gln Asp Ala Arg Asp Leu Tyr Asp Ala Gly Val Lys Arg Lys Gly Thr
           195                 200                 205
   Asp Val Pro Lys Trp Ile Ser Ile Met Thr Glu Arg Ser Val Pro His
       210                 215                 220
   Leu Gln Lys Val Phe Asp Arg Tyr Lys Ser Tyr Ser Pro Tyr Asp Met
   225                 230                 235                 240
   Leu Glu Ser Ile Arg Lys Glu Val Lys Gly Asp Leu Glu Asn Ala Phe
                   245                 250                 255
   Leu Asn Leu Val Gln Cys Ile Gln Asn Lys Pro Leu Tyr Phe Ala Asp
               260                 265                 270
   Arg Leu Tyr Asp Ser Met Lys Gly Lys Gly Thr Arg Asp Lys Val Leu
           275                 280                 285
   Ile Arg Ile Met Val Ser Arg Ser Glu Val Asp Met Leu Lys Ile Arg
       290                 295                 300
   Ser Glu Phe Lys Arg Lys Tyr Gly Lys Ser Leu Tyr Tyr Tyr Ile Gln
   305                 310                 315                 320
   Gln Asp Thr Lys Gly Asp Tyr Gln Lys Ala Leu Leu Tyr Leu Cys Gly
                   325                 330                 335
   Gly Asp Asp
<210> 6
   <211> 24
   <212> DNA
   <213> Séquence artificielle
<220>
   <223> Amorce PCR
<400> 6
   gattctgcct acgaacgacg actt 24
<210> 7
   <211> 23
   <212> DNA
   <213> Séquence artificielle
<220>
   <223> Amorce PCR
<400> 7
   gggagatgat ccgttgatgc gag 23
<210> 8
   <211> 97
   <212> DNA
   <213> Séquence artificielle
<220>
   <223> Séquence de l'aptamère ER2
<400> 8
   gggagatgat ccgttgatgc gagcagagcc gctggcgact tattccaaca gtcgccccca     60
   caacccttgt cccaagtcgt cgttcgtagg cagaatc                              97
<210> 9
   <211> 21
   <212> RNA
   <213> Séquence artificielle
<220>
   <223> siRNA anti-annexine 2
<400> 9
   ucauccacac cuuuggucuu u 21
<210> 10
   <211> 21
   <212> RNA
   <213> Séquence artificielle
<220>
   <223> siRNA anti-annexine 2
<400> 10
   ucagcaucaa aguuaguauu u 21
<210> 11
   <211> 97
   <212> DNA
   <213> Séquence artificielle
<220>
   <223> Séquence de l'aptamère contrôle
<400> 11
   gggagatgat ccgttgatgc gaggatccct acgacctcgt agcacacaca taggtgcact     60
   cacccggctg accaagtcgt cgttcgtagg cagaatc                              97

## Description

### Domaine de l'invention

La présente invention concerne un ligand spécifique de l'annexine 2, ainsi que son utilisation diagnostique et thérapeutique, ou son utilisation pour la détection *in vitro* de l'annexine 2 dans un échantillon biologique.

### Arrière-plan technique

L'annexine 2, également nommée annexine A2, annexine II, lipocortine II, chaîne lourde de la calpactine I, chromobindine-8, p. 36 ou protéine placentaire anticoagulante IV, est une protéine de 38 kDa qui fait partie de la famille des annexines. Les annexines sont des protéines qui possèdent une double localisation cellulaire : cytosolique et membranaire. Elle est codée chez l'homme par le gène *ANXA2.* L'annexine 2 est structurée en 2 domaines : le coeur qui contient les sites de liaison au Ca²⁺ responsables de la liaison aux membranes, et le domaine N-terminal qui possède le site de liaison à la protéine S100A10 avec laquelle elle forme un tétramère.

L'annexine 2 est impliquée dans de nombreuses fonctions : l'angiogénèse, le processus métastatique, le transport du cholestérol, l'infection par certains virus, les phénomènes d'exocytose et d'endocytose, la régulation de l'action du plasminogène, la fibrinolyse, la formation de canaux ioniques, les interactions membrane-cytosquelette, la formation de jonctions intercellulaires (pour une revue voir Hajjar, K. A. & S. Krishnan (1999). Trends in Cardiovascular Medicine 9(5): 128-138).

Sur le plan pathologique, la surexpression de l'annexine A2 a été retrouvée dans plusieurs cancers humains, incluant, de manière non restrictive, les gliomes de haut grade, les leucémies aiguës promyélocytaires, le cancer colorectal, le cancer du pancréas, les carcinomes à cellules rénales, les carcinomes hépatocellulaires, les carcinomes à cellules squameuses, le cancer de la prostate, et le cancer du poumon. La surexpression de l'annexine 2 a d'ailleurs été corrélée à un mauvais pronostic dans le cancer colorectal (Emoto et al. (2001). Cancer 92:1419-26) et à un risque de récidive après chirurgie chez les patients atteints du cancer du pancréas (Takano et al. (2008). Ann Surg Oncol 15:3157-68).

L'implication de l'annexine 2 a été rapportée dans plusieurs maladies. Ainsi, il apparaît que des niveaux anormalement élevés d'expression d'annexine 2 sur les cellules de leucémie promyélocytaire aiguë augmentent la production de plasmine, une protéine fibrinolytique, ce qui contribuerait aux complications hémorragiques observées dans cette leucémie (Menell et al. (1999) New England Journal of Medicine 340:994-1004; Hajjar & Krishnan (1999) Trends Cardiovasc. Med. 9:128-138 Stein et al. (2009) Best Pract. Res. Clin. Haematol. 22:153-63.

Il a également été rapporté que l'annexine 2 favoriserait l'angiogenèse, la progression tumorale, ainsi que les métastases de certains cancers (Yusuke, S. et al. (2008) Journal of Cellular Biochemistry 105(2): 370-380).

Par ailleurs, l'annexine 2 serait également impliquée dans le risque accru de thrombose artérielle et veineuse que présentent les patients porteurs d'anticorps anti-phospholipides (APLA) (Zhang & McCrae (2005) Blood 105:1964-1969). L'annexine 2 favoriserait aussi l'infection des cellules par certains virus.

L'annexine 2 est donc une cible thérapeutique prometteuse ainsi qu'un marqueur diagnostique intéressant, et il serait avantageux de disposer de ligands spécifiques de cette protéine, que ce soit pour bloquer ses fonctions pathologiques ou pour la détecter.

A ce titre, il a été montré que plusieurs ligands protéiques de l'annexine 2 avaient une action thérapeutique. Ainsi, il a été montré chez la souris qu'un anticorps monoclonal dirigé contre l'annexine 2 permettait de réduire de 70% la croissance tumorale dans un modèle murin de carcinome pulmonaire de Lewis (Sharma et al. (2006) Exp. Mol. Pathol. 81:136145). De même, il a été démontré que l'effet anti-angiogénique du polypeptide TM601, forme synthétique de la chlorotoxine, serait lié à son interaction avec l'annexine 2 (Kesavan et al. (2010) J. Biol. Chem. 285:4366-4374 ; Lima et al. (2010). J Cell Physiol 225:855-864). Par ailleurs, il a été montré que le fragment Fab d'un anticorps monoclonal anti-annexine 2 bloque l'activation endothéliale provoquée par des APLA (Zhang & McCrae (2005) Blood 105:1964-1969).

Toutefois, les ligands de l'annexine 2 identifiés jusqu'à ce jour présentent des limitations, notamment en terme de coût de synthèse, d'immunogénicité ou d'affinité pour leur cible.

### Résumé de l'invention

La présente invention découle de la mise en évidence inattendue, par les inventeurs, d'un aptamère, de nature nucléotidique, reconnaissant spécifiquement l'annexine 2 à la surface de cellules, avec une constante de dissociation de l'ordre du nanomolaire.

Ainsi, la présente invention concerne un aptamère comprenant un acide nucléique capable de reconnaître spécifiquement l'annexine 2 à la surface d'une cellule.

Plus particulièrement, l'invention concerne un aptamère comprenant, ou consistant en, un acide nucléique comprenant, ou constitué de :
- la séquence GGAACGCAAGAACUGAGGCCAUGAGGCGCCUUCCCUUGCUCA GGACGC (SEQ ID NO : 1), ou
- la séquence AGCUAGGCCGCAAGGUGCCUCAACGCCAUCUGAGUGCCGACC CGAUCGC (SEQ ID NO : 2), ou
- une séquence comprenant, ou constituée d'au moins 15 nucléotides consécutifs d'un polynucléotide ayant au moins 60% d'identité avec SEQ ID NO : 1 ou SEQ ID NO : 2, sous réserve qu'un acide nucléique constitué de cette séquence se lie à l'annexine 2.

Dans un mode de réalisation particulier, l'invention concerne l'aptamère selon l'invention, pour son utilisation à titre de médicament ou d'agent diagnostique.

La présente invention concerne également une composition pharmaceutique ou diagnostique comprenant, à titre de substance active, au moins un aptamère selon l'invention, en association avec au moins un véhicule pharmaceutiquement acceptable.

A ce titre, l'invention concerne également une méthode *in vitro* de diagnostic d'un cancer chez un individu, comprenant les étapes suivantes :
- mettre en contact l'échantillon biologique avec un aptamère selon l'invention ;
- déterminer la quantité d'aptamère fixé dans l'échantillon ;
- éventuellement comparer la quantité d'aptamère fixé dans l'échantillon à au moins une valeur prédéterminée
- en déduire si l'individu est atteint d'un cancer.

La présente invention concerne également une méthode de diagnostic d'un cancer chez un individu, comprenant les étapes suivantes :
- administrer un aptamère selon l'invention à l'individu ;
- détecter, quantifier et/ou localiser l'aptamère dans l'individu ou une partie de l'individu ;
- en déduire si l'individu est atteint d'un cancer.

La présente invention concerne également l'utilisation *in vitro* d'un aptamère selon l'invention, pour détecter la présence d'annexine 2, ou déterminer la quantité d'annexine 2, présente dans un échantillon biologique.

La présente invention concerne une méthode *in vitro* de détection ou détermination de la quantité d'annexine 2 dans un échantillon biologique, comprenant les étapes suivantes :
- mettre en contact l'échantillon biologique avec un aptamère selon l'invention ;
- quantifier, ou détecter la présence ou l'absence d'aptamère fixé dans l'échantillon ;
- en déduire la quantité, ou la présence ou l'absence d'annexine 2 dans l'échantillon.

La présente invention concerne également une méthode de détection, de quantification ou de localisation d'annexine 2, dans un individu ou une partie d'un individu, comprenant les étapes suivantes :
- administrer un aptamère ou une composition diagnostique tel que défini ci-dessus à l'individu ;
- détecter, quantifier et/ou localiser l'aptamère dans l'individu ou une partie de l'individu ;
- en déduire la présence ou l'absence, la quantité, et/ou la localisation d'annexine 2 dans l'individu ou la partie de l'individu.

La présente invention concerne également l'utilisation d'un aptamère tel que défini ci-dessus, pour le criblage de ligands de l'annexine 2.

La présente invention concerne également une méthode de criblage de ligands de l'annexine 2 comprenant les étapes suivantes :
- la mise en présence d'annexine 2 et, concomitamment ou successivement, d'un ligand à cribler et d'un aptamère tel que défini ci-dessus ;
- déterminer la quantité d'aptamère fixé à l'annexine 2 ;
- en déduire si le ligand est un ligand de l'annexine 2.

### Description détaillée de l'invention

Comme on l'entend ici, un « aptamère » désigne un composé, comprenant au moins un acide nucléique, susceptible de se lier de manière spécifique à une cible, notamment de nature protéique, par l'intermédiaire de l'acide nucléique. Un aptamère est dit se lier de manière spécifique à une cible lorsqu'il ne présente essentiellement pas d'affinité pour un composé sans rapport structural avec la cible. De préférence, dans le cas d'une cible protéique, un composé protéique est dit sans rapport structural avec la cible selon l'invention, lorsque l'identité de séquence entre la cible et le composé est inférieure à 60%, de préférence inférieure à 70%, de préférence encore inférieure à 80%. De préférence, selon l'invention, un aptamère est dit ne présenter essentiellement pas d'affinité pour un composé selon l'invention, notamment lorsque la constante de dissociation de l'aptamère vis-à-vis du composé est supérieure à 10⁻⁶ mol/l, de préférence supérieure à 10⁻⁷ mol/l. La constante de dissociation peut notamment être déterminée, dans des conditions standards, à l'aide des représentations de Scatchard et de Lineweaver Burk bien connues de l'homme du métier.

De manière avantageuse, l'aptamère selon l'invention est spécifique de l'annexine 2, en particulier de l'annexine 2 humaine, notamment lorsque l'annexine 2 est exprimée à la surface d'une cellule. L'annexine 2, également nommée annexine A2, annexine II, lipocortine II, chaîne lourde de la calpactine I, chromobindine-8, p. 36 ou protéine placentaire anticoagulante IV, est bien connue de l'homme du métier. L'annexine 2 humaine est notamment décrite sous la référence AAH52567.1 dans la base de données *GenBank.* A titre d'exemple, l'annexine 2 humaine est représentée par la séquence SEQ ID NO : 5.

L'aptamère selon l'invention peut également comprendre au moins un groupement additionnel en plus de l'acide nucléique. Ainsi, l'acide nucléique selon l'invention peut être lié à au moins un groupement additionnel. Par ailleurs, de manière préférentielle, l'aptamère selon l'invention est constitué de l'acide nucléique selon l'invention et d'au moins un groupement additionnel selon l'invention.

Le groupement additionnel selon l'invention peut être de tout type et de toute nature. Le groupement additionnel selon l'invention peut ainsi être un radioisotope, une molécule organique comprenant 100 atomes de carbone au plus, une nanoparticule, notamment une micelle, une protéine, notamment une glycoprotéine, un glucide, un lipide, ou encore un polynucléotide. Selon l'invention, on préfère toutefois que le groupement additionnel selon l'invention soit sélectionné dans le groupe constitué d'un marqueur détectable, d'un composé pharmacologique, et d'un composé susceptible de modifier les caractéristiques pharmacocinétiques d'un acide nucléique auquel il est lié, tel que le polyéthylène glycol (PEG).

Le marqueur détectable selon l'invention peut être de tout type, il peut notamment s'agir d'un fluorophore, par exemple la fluorescéine ou la luciférase ; d'un radioisotope, notamment adapté à la scintigraphie, par exemple ^{99m}Tc ; d'une étiquette reconnaissable par un anticorps, par exemple la protéine c-Myc ; d'une étiquette d'affinité, par exemple la biotine ; d'une enzyme, par exemple la peroxydase de raifort.

Le composé pharmacologique selon l'invention peut également être de tout type, il peut notamment s'agir d'un agent de chimiothérapie anticancéreuse, tel qu'un agent cytostatique ou cytolytique. Le composé pharmacologique selon l'invention peut également être de toute nature, il peut notamment s'agir d'un dérivé du platine, d'une molécule organique comprenant moins de 100 atomes de carbone, d'un peptide, d'un analogue de nucléotide, d'une toxine, d'un ARN interférent, ou d'un oligonucleotide antisens.

De préférence, l'acide nucléique selon l'invention est de l'ARN. Comme cela apparaîtra de manière claire à l'homme du métier, il est tout particulièrement préféré que l'acide nucléique selon l'invention soit monobrin. Il est également tout particulièrement préféré que l'acide nucléique selon l'invention ait une structure tridimensionnelle lui permettant de se lier spécifiquement à l'annexine 2. Par ailleurs, le squelette ou le ribose de l'acide nucléique selon l'invention peut être modifié, en totalité ou en partie, notamment pour le rendre résistant à une dégradation hydrolytique, en particulier du fait de l'action de nucléase, notamment lorsque l'acide nucléique est de l'ARN. De telles modifications sont bien connues de l'homme du métier et recouvrent notamment les modifications de la fonction OH sur le carbone en position 2' du ribose par méthylation, ou la substitution de cette fonction OH par un groupement amino ou par un halogène, notamment par le fluor, ainsi que le recours à un squelette phosphorothioate, ou à des structure de type acide nucléique bloqué (*Locked Nucleic Acid*, LNA) ou acide nucléique peptidique (*Peptide Nucleic Acid*, PNA). Ainsi, de manière préférée, l'acide nucléique selon l'invention est un ARN dont les riboses des nucléotides pyrimidines portent un atome de fluor sur le carbone en position 2', les riboses des nucléotides puriques pouvant être inchangés.

Une séquence ayant au moins 60% d'identité en nucléotides avec SEQ ID NO : 1 ou SEQ ID NO : 2 selon l'invention, diffère notamment de SEQ ID NO : 1 ou 2 par l'insertion, la suppression ou la substitution d'au moins un nucléotide. Comme on l'entend ici, le pourcentage d'identité entre deux séquences est défini comme le nombre de positions pour lesquelles les bases sont identiques lorsque les séquences sont alignées de manière optimale, divisé par le nombre total de bases de la plus grande des deux séquences. Deux séquences sont dites alignées de manière optimale lorsque le pourcentage d'identité est maximal. Par ailleurs, comme cela apparaitra de manière claire à l'homme du métier, il peut être nécessaire de faire appel à des ajouts de positions lacunaires (des « *gaps* ») de manière à obtenir un alignement optimal entre les deux séquences.

Un acide nucléique est dit se lier à l'annexine 2, si la constante de dissociation de l'acide nucléique vis-à-vis de l'annexine 2, notamment humaine, de préférence exprimée par une cellule, en particulier une cellule de lignée cellulaire CHO, HEK293, MDA MB 231, MCF-7, A431, PC12 MEN2A, U87, 4T1, ou EMT6, comme cela est illustré dans les exemples, est inférieure à 10⁻⁶ mol/l, de préférence inférieure à 10⁻⁷ mol/l.

Comme cela apparaîtra clairement à l'homme du métier, lorsque l'aptamère selon l'invention comprend un acide nucléique selon l'invention, il peut également comprendre d'autres acides nucléiques. En revanche, lorsque l'aptamère selon l'invention est constitué de l'acide nucléique selon l'invention, il ne comprend pas d'autres acides nucléiques. De manière similaire, lorsque l'acide nucléique selon l'invention comprend une séquence, il peut également comprendre des séquences additionnelles s'étendant du côté 5' et/ou 3' de la séquence en question. En revanche, lorsque l'acide nucléique selon l'invention est constitué d'une séquence, il ne comprend pas de séquences additionnelles en plus de la séquence en question.

Une séquence comprenant SEQ ID NO : 1 ou 2 selon l'invention peut notamment comprendre des séquences du côté 5' et/ou 3' visant à structurer l'acide nucléique. On préfère ainsi que l'acide nucléique selon l'invention comprenne, ou soit constitué de SEQ ID NO : 3 ou 4, qui comprennent respectivement SEQ ID NO : 1 et 2. Dans ce cadre, l'invention concerne alors également, en particulier, un acide nucléique comprenant, ou constitué d'au moins 15 nucléotides consécutifs d'une séquence ayant au moins 60% d'identité avec SEQ ID NO : 3 ou SEQ ID NO : 4, sous réserve qu'un acide nucléique constitué de cette séquence présente se lie à l'annexine 2.

De préférence, la séquence comprenant, ou constituée d'au moins 15 nucléotides consécutifs d'une séquence ayant au moins 60% d'identité avec SEQ ID NO : 1, 2, 3 ou 4 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 60% d'identité avec SEQ ID NO : 1, 2, 3 ou 4.

De préférence également, la séquence comprenant, ou constituée d'au moins 15 nucléotides consécutifs d'une séquence ayant au moins 60% d'identité avec SEQ ID NO : 1, 2, 3 ou 4 selon l'invention, comprend ou est constituée d'au moins 15 nucléotides consécutif d'une séquence ayant au moins 65%, 70%, 75%, 80%, 85%, 90%, 95%, ou 100%, d'identité avec SEQ ID NO : 1, 2, 3 ou 4 selon l'invention.

De manière plus préférée, la séquence comprenant, ou constituée d'au moins 15 nucléotides consécutifs d'une séquence ayant au moins 60% d'identité avec SEQ ID NO : 1, 2, 3 ou 4 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 80% d'identité avec SEQ ID NO : 1, 2, 3 ou 4.

De manière davantage préférée, la séquence comprenant, ou constituée d'au moins 15 nucléotides consécutifs d'une séquence ayant au moins 60% d'identité avec SEQ ID NO : 1, 2, 3 ou 4 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 85% d'identité avec SEQ ID NO : 1, 2, 3 ou 4.

De manière encore plus préférée, la séquence comprenant, ou constituée d'au moins 15 nucléotides consécutifs d'une séquence ayant au moins 60% d'identité avec SEQ ID NO : 1, 2, 3 ou 4 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 90% d'identité avec SEQ ID NO : 1, 2, 3 ou 4.

De manière particulièrement préférée, la séquence comprenant, ou constituée d'au moins 15 nucléotides consécutifs d'une séquence ayant au moins 60% d'identité avec SEQ ID NO : 1, 2, 3 ou 4 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 95% d'identité avec SEQ ID NO : 1, 2, 3 ou 4.

Par ailleurs, de manière alternative, la séquence comprenant, ou constituée d'au moins 15 nucléotides consécutifs d'une séquence ayant au moins 60% d'identité avec SEQ ID NO : 1, 2, 3, ou 4 selon l'invention, peut être constituée d'une séquence de 95 nucléotides présentant au moins 60%, 65%, 70%, 75%, 80%, 85%, 90% ou 95% d'identité avec SEQ ID NO : 3 et un acide nucléique constitué de cette séquence est susceptible d'adopter la structure de formule (I) suivante : 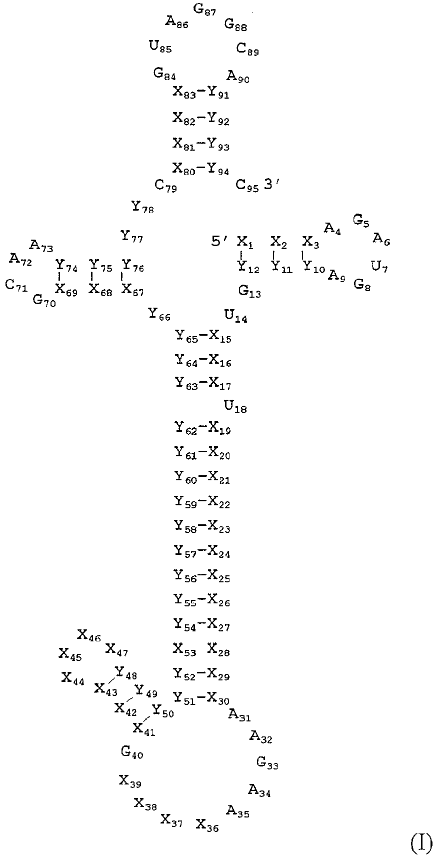 dans laquelle :
- A, C, G, U sont les ribonucléotides Adenosine, Cytidine, Guanosine, et Uridine ;
- chacun des X et des Y, identiques ou différents, représente A, C, G ou U ;
- chacune des paires X-Y ou Y-X, identiques ou différentes, représentent des paires A-U, U-A, G-C, C-G, G-U ou U-G ;
- de préférence (i) X₃₆, X₃₇, X₃₈, X₃₉ sont capables de former des paires A-U, U-A, G-C, C-G, G-U ou U-G respectivement avec Y₆₆, Y₆₅, Y₆₄ et Y₆₃, et (ii) X₄₄, X₄₅, X₄₆, X₄₇ sont capables de former des paires respectivement avec Y₇₈, Y₇₇, Y₇₆ et Y₇₅.

De manière alternative également, la séquence comprenant, ou constituée d'au moins 15 nucléotides consécutifs d'une séquence ayant au moins 60% d'identité avec SEQ ID NO : 1, 2, 3, ou 4 selon l'invention, peut être constituée d'une séquence de 96 nucléotides présentant au moins 60%, 65%, 70%, 75%, 80%, 85%, 90% ou 95% d'identité avec SEQ ID NO : 4 et un acide nucléique constitué de cette séquence est susceptible d'adopter la structure de formule (II) suivante : 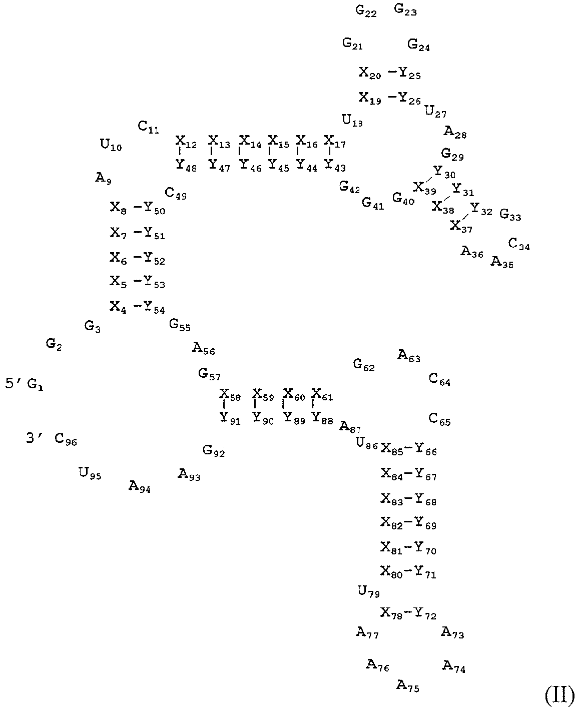 dans laquelle :
- A, C, G, U sont les ribonucléotides Adenosine, Cytidine, Guanosine, et Uridine ;
- chacun des X et des Y, identiques ou différents, représente A, C, G ou U ;
- chacune des paires X-Y ou Y-X, identiques ou différentes, représentent des paires A-U, U-A, G-C, C-G, G-U ou U-G.

L'homme du métier peut aisément déterminer la structure secondaire susceptible d'être adoptée par un acide nucléique ayant une séquence selon l'invention, par exemple à l'aide d'algorithmes ou de logiciels de modélisation bien connus de l'homme du métier, tels que *mfold* (version 3.4) décrit dans Nucleic Acids Res. (2003) 31:3406-15 et *vsfold5 RNA Pseudoknot Prediction* décrit dans PLoS One (2007), 2:905. A ce titre, les inventeurs ont établi, à l'aide de tels outils de modélisation, que l'aptamère ACE8 de séquence SEQ ID NO : 3, qui est décrit dans les Exemples, adopte une structure de formule (I) et que l'aptamère ER4, de séquence SEQ ID NO : 4, qui est décrit dans les Exemples, adopte une structure de formule (II).

Lorsque l'aptamère selon l'invention est utilisé à titre de médicament ou est compris dans une composition pharmaceutique, il est notamment utile pour la prévention ou le traitement des cancers, notamment en inhibant l'angiogenèse, pour la prévention ou le traitement des maladies impliquant une néovascularisation oculaire, telles que la dégénérescence maculaire liée à l'âge et la rétinopathie diabétique, pour la prévention ou le traitement de la thrombose artérielle ou veineuse chez les patients porteurs d'anticorps anti-phospholipides (APLA), pour la prévention ou le traitement des hémorragies chez les patients atteints de leucémie promyélocytaire aiguë, ainsi que pour la prévention ou le traitement des infections virales.

Lorsque l'aptamère selon l'invention est utilisé à titre d'agent diagnostique ou est compris dans une composition diagnostique, il est notamment utile pour le diagnostic des cancers.

De préférence, dans les méthodes de détection ou de diagnostic selon l'invention la détection d'aptamère fixé dans l'échantillon est effectuée par mise en oeuvre d'une réaction de polymérisation en chaîne (PCR) destinée à amplifier l'aptamère. De préférence, lorsque l'acide nucléique selon l'invention comprend SEQ ID NO : 3 ou 4, la PCR est mise en oeuvre à l'aide du couple d'amorces de séquences GATTCTGCCTACGAACGACGACTT (SEQ ID NO : 6) et GGGAGATGATCCGTTGATGCGAG (SEQ ID NO : 7). Par ailleurs, l'invention concerne également un acide nucléique comprenant ou constitué de SEQ ID NO : 6 ou de SEQ ID NO : 7. De préférence également, dans les méthodes de détection ou de diagnostic selon l'invention, lorsque l'aptamère est administré à un individu, on préfère que l'aptamère soit détectable par des méthodes d'imagerie *in vivo*, notamment externes, telles que l'imagerie fluorescente planaire ou tridimensionnelle (3D), ou internes, telles que l'endoscopie par exemple.

### Description des figures

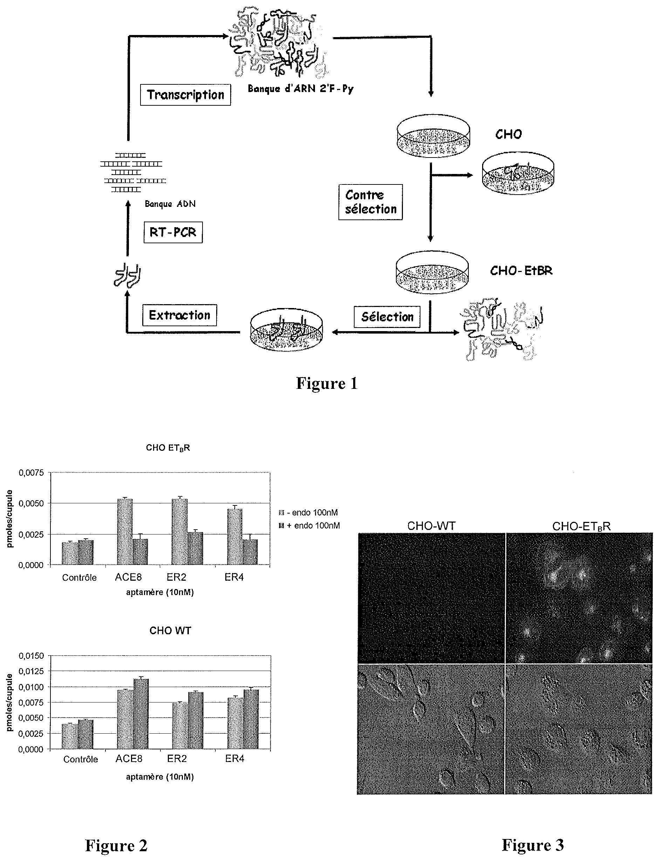

### Figure 1 : schéma du protocole de sélection

La figure 1 représente le protocole de sélection des aptamères utilisé par les inventeurs pour obtenir l'aptamère ACE8.

### Figures 2 et 3 : Effet de l'endothéline sur la liaison des aptamères

La figure 2 représente la fixation des aptamères ACE8, ER2 et ER4 sur les cellules CHO-ET_{B}R (graphe du haut) et CHO non transformées (CHO-WT, graphe du bas) en présence ou non d'endothéline. Les cellules CHO-ET_{B}R ont été pré-incubées dans des cupules pendant 30 minutes (colonnes sombres) ou non (colonnes claires) avec 100nM d'endothéline. Puis les aptamères radiomarqués sont ajoutés à une concentration de 10 nM pendant 30 minutes. Après plusieurs lavages, la radioactivité fixée aux cellules est comptée et la quantité d'aptamères fixés est déterminée (en pmoles/cupule).

La figure 3 permet de visualiser l'endothéline sur CHO-ETBR et CHO-WT. Les cellules sont incubées pendant 30 min avec de l'endothéline-1-FAM à une concentration de 20 nM. Après deux lavages, les cellules sont visualisées par microscopie fluorescente (photographies du haut) et montre une forte internalisation de l'endothéline. Les photographies du bas représentent les champs clairs correspondant aux champs fluorescents.

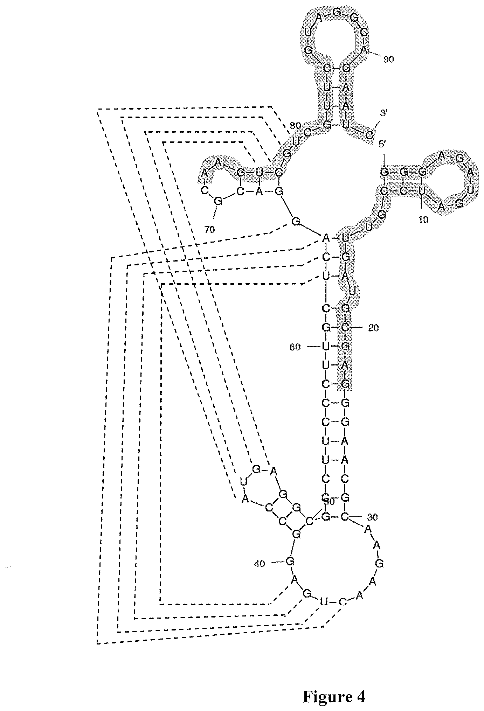

### Figure 4 : Prédiction de structure de l'aptamère ACE8

La figure 4 représente la structure secondaire de l'aptamère ACE8 prédite à l'aide du programme mfold (version 3.2, http://mfold.bioinfo.rpi.edu/cgi-bin/rna-form1.cgi). Sur cette structure, les séquences fixes permettant une amplification par PCR ont été surlignées en gris. La formation potentielle de pseudo-noeuds prédite par le programme Vsfold5 a été rajoutée (Vsfold5 *pseudoknot prediction program*, http://www.rna.it-chiba.ac.jp/∼vsfold/vsfold5/).

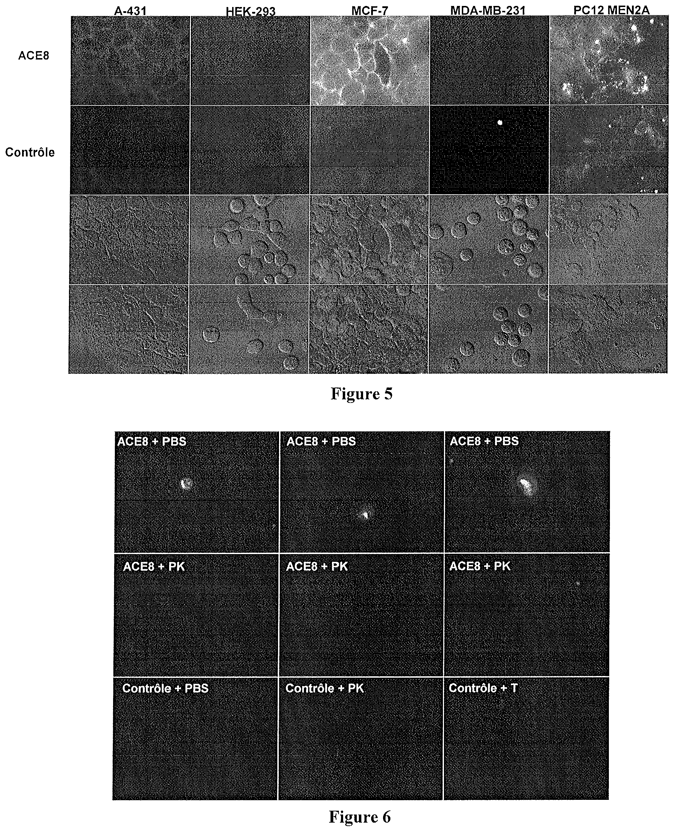

### Figure 5 : Visualisation de l'aptamère ACE8 sur différents types cellulaires

La figure 5 représente des photographies prises en microscopie à fluorescence (photographies du haut) et les champs clairs correspondants (photographies du bas) pour des cellules A-431, HEK-293, MCF-7, MDA-MB-231 et PC12 MEN2A mises en contact avec l'aptamère ACE8 ou la séquence contrôle marqué à la phycoérythrine. L'aptamère ou la séquence contrôle est incubé sur les cellules pendant 15 min à une concentration de 50 nM. Après 2 lavages, les cellules sont visualisées au microscope à fluorescence. Le temps d'exposition a été fixé à 200 ms pour toutes les expériences.

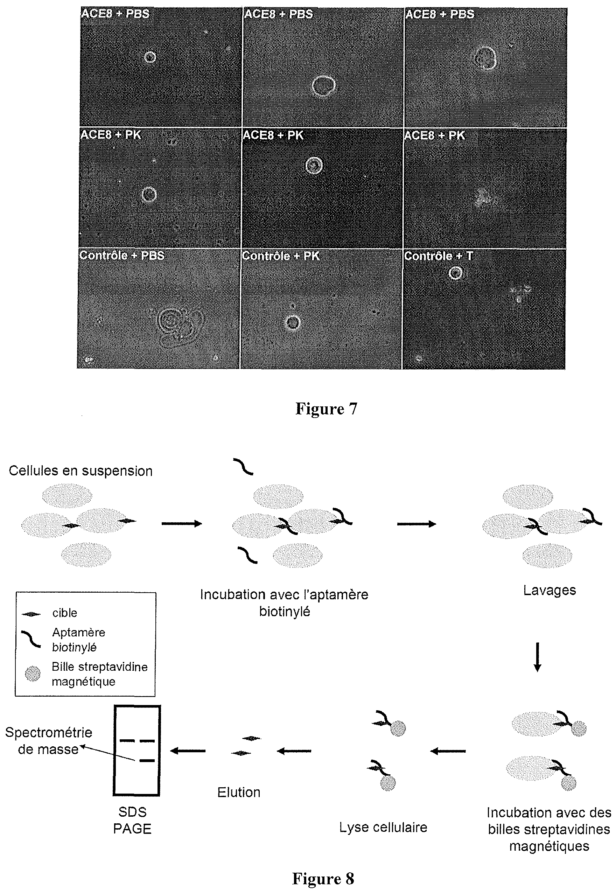

### Figures 6 et 7 : Fixation de l'aptamère ACE8 sur des cellules MCF-7 en suspension

La figure 6 représente des photographies prises en microscopie à fluorescence de cellules MCF-7 incubées en présence de l'aptamère ACE8 ou d'une séquence contrôle couplée à la phycoérythrine et en présence de protéinase K (+PK), de trypsine (+T) ou de PBS. Brièvement, les cellules MCF-7 sont détachées avec une solution de PBS-EDTA 5 mM. Les cellules en suspension sont pré-incubées avec de la protéinase K (+PK), de la trypsine (+T) ou du PBS pendant 10 min à 37°C. 1 mL de sérum de veau foetal inactivé (SVFi) sont ajoutés pour inhiber les enzymes. Les cellules sont alors incubées pendant 30 min avec l'aptamère ACE8 ou la séquence contrôle couplés à la phycoérythrine et lavées deux fois. Les cellules sont observées entre lame et lamelle au microscope à épifluorescence. La figure 7 représente les champs clairs correspondants aux champs fluorescents.

### Figure 8 : Protocole d'identification de la cible de l'aptmère ACE8

La figure 8 représente le protocole d'identification de la cible de l'aptamère ACE8. Brièvement, l'aptamère biotinylé est incubé sur les cellules MCF-7 en suspension. Après plusieurs lavages, des billes magnétiques couplées à la streptavidine sont ajoutées. Seules les cellules qui ont fixé l'aptamère ACE8 biotinylé vont être retenues sur les billes. Les cellules sont alors lysées et les protéines retenues par l'aptamère ACE8 sont éluées. Les protéines sont alors déposées sur gel SDS-PAGE et les bandes spécifiques sont analysées par spectrométrie de masse.

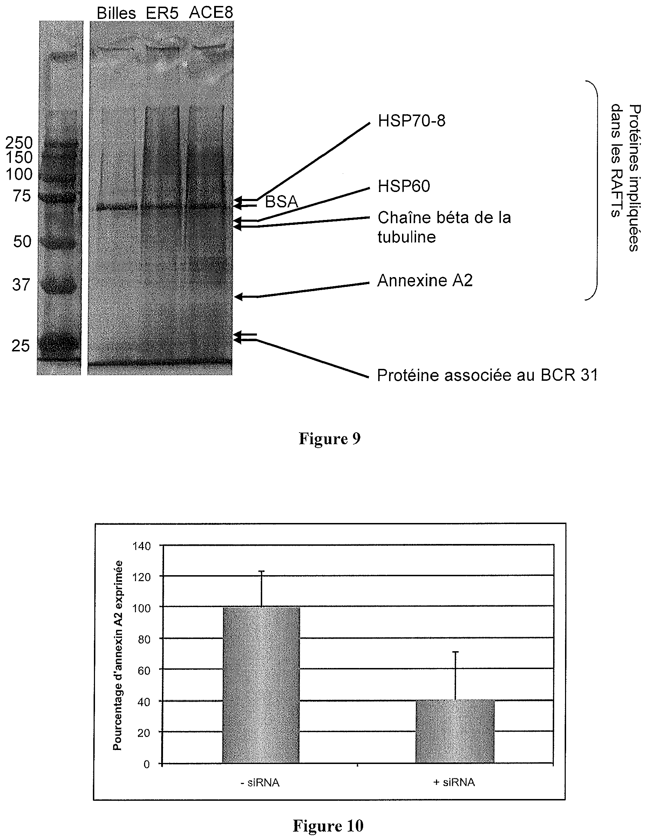

### Figure 9 : Migration des protéines par électrophorèse SDS-PAGE

La figure 9 est une photographie d'un gel SDS-PAGE sur lequel sont mis à migrer les éluats provenant de billes mises directement en contact avec les cellules (billes), d'un oligonucléotide contrôle (Ctrl), ou de l'aptamère ACE8. Les principales bandes correspondant à l'éluat provenant de l'aptamère sont indiquées. La bande correspondant à l'Albumine Sérique Bovine (BSA), utilisée comme compétiteur non spécifique, est également représentée. Brièvement, les protéines sont déposées sur un gel SDS PAGE 10%. Le gel est ensuite coloré à l'aide du kit protéosilver silver stain kit (sigma). Les bandes intéressantes sont découpées, décolorées avec le même kit et analysées en spectrométrie de masse.

### Figure 10 : Etude de l'effet de siRNA sur l'expression de l'annexine 2 par qPCR dans les cellules MCF-7

La figure 10 représente la quantité d'ARNm codant l'annexine 2 (axe des ordonnées, en pourcentage de la quantité exprimée en l'absence de siRNA) exprimée par des cellules MCF-7 en présence d'un siRNA ciblant (+siRNA) ou non (-siRNA) l'ARNm de l'annexine 2. Les cellules sont transfectées ou non avec un siRNA ciblant l'annexine 2 en présence de lipofectamine. 72 h après, l'ARN total des cellules MCF-7 est extrait, rétro-transcrit et une qPCR est réalisé en utilisant des amorces spécifiques de l'annexine 2 et du gène P0 pour normaliser les résultats.

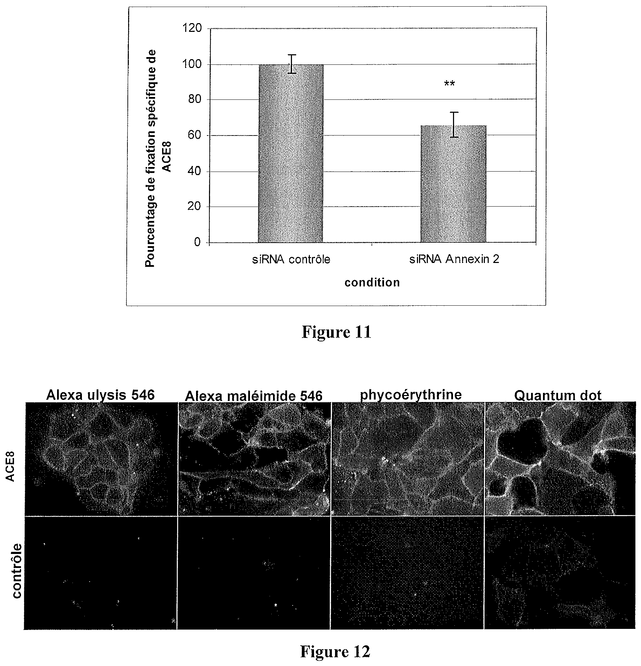

### Figure 11 : Diminution de la fixation de l'aptamère ACE8 après traitement avec un siRNA ciblant l'annexine 2

La figure 11 représente la quantité d'aptamères ACE8 (axe des ordonnées, en pourcentage de la quantité fixée en l'absence de siRNA) fixée à la surface de cellules MCF-7 en présence d'un siRNA ciblant (siRNA Annexine 2) ou non (siRNA contrôle) l'ARNm de l'annexine 2. Les cellules MCF-7 sont transfectées avec un siRNA ciblant l'annexine 2 ou avec un siRNA contrôle en présence de lipofectamine. 72 h après, l'aptamère ACE8 radiomarqué ou une séquence contôle radiomarquée est incubé à une concentration de 10 nM pendant 15 min. La radioactivité est alors comptée. ** correspond à un écart significatif de la fixation de ACE8 entre les cellules transfectées par le siRNA ciblant l'annexine 2 et les cellules transfectées par le siRNA contrôle mesuré par un test de student avec P<0.01.

### Figure 12 : Utilisation des aptamères en microscopie

La figure 12 représente des photographies en microscopie à fluorescence de l'aptamère ACE8 ou d'une séquence contrôle couplés directement à l'Alexa ulysis 546, ou indirectement, à l'Alexa Fluor 546 maléimide, en les ayant préalablement couplés à un groupement SH, ou à la Phycoérythrine et à des Quantum Dots, en les ayant préalablement couplés à une biotine, et mis en présence de cellules MCF-7. Les aptamères marqués sont incubés pendant 15 min à une concentration de 50 nM sur les cellules MCF-7. Après 2 lavages, les cellules sont observées au microscope à fluorescence.

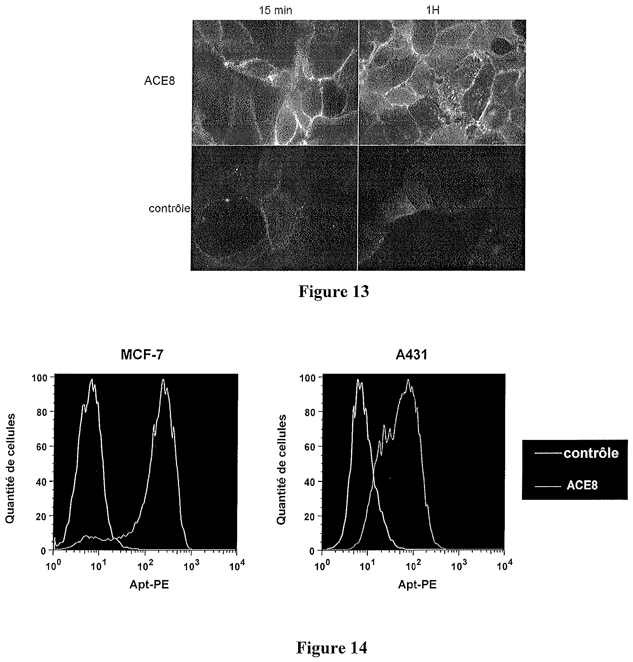

### Figure 13 : Ciblage de nanoparticules à l'aide de l'aptamère ACE8

La figure 13 représente des photographies en microscopie à fluorescence de cellules MCF-7 mises en présence de l'aptamère ACE8 ou d'une séquence contrôle couplés à des Quantum Dots (QDs) pendant 15 min ou 1 h. L'aptamère permet un ciblage rapide des QDs à la membrane des cellules MCF-7 puis une partie est internalisée en moins d'une heure. Une séquence contrôle couplée à des QDs ne montre aucun marquage des cellules.

### Figure 14 : Utilisation de l'aptamère ACE8 en cytométrie de flux (FACS)

Les cellules MCF-7 ou A431 sont incubées avec l'aptamère ACE8 ou la séquence contrôle marqués à la phycoérythrine à une concentration de 5 nM pendant 30 min. Les cellules sont alors lavées et analysées par cytométrie de flux. La figure 14 représente la quantité de cellules triées (axe des ordonnées) MCF-7 (graphe de gauche) et A431 (graphe de droite) en fonction de la fluorescence émise par l'aptamère ACE8 ou la séquence contrôle (axe des abscisses, unités arbitraires).

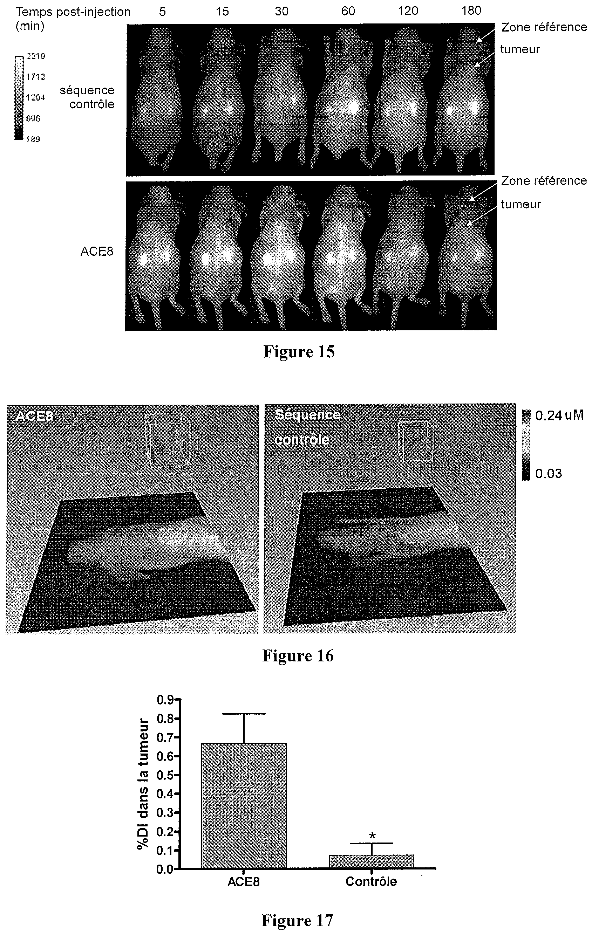

### Figure 15 : Utilisation de l'aptamère ACE8 en imagerie in vivo fluorescente planaire

L'aptamère ACE8 ou une séquence contrôle marqués avec de l'alexaFluor680 ont été injectés dans des souris développant des tumeurs suite à une injection sous cutanée de cellules MCF-7. La Figure 15 représente leur biodistribution mesurée à l'aide de photographies obtenues à différents temps par imagerie fluorescente planaire à l'aide d'un appareil TomoFluo3D développé par le CEA-LETI. La tumeur ainsi que la zone de référence qui permet de mesurer le rapport signal dans la tumeur/référence interne sont indiquées par des flèches blanches.

### Figures 16 et 17 : Utilisation de l'aptamère ACE8 en imagerie in vivo fluorescente 3D

L'aptamère ACE8 ou une séquence contrôle marqués avec de l'alexaFluor680 ont été injectés dans des souris développant des tumeurs suite à une injection sous cutanée de cellules MCF-7. Trois heures post-injection, le signal fluorescent dans la tumeur a été mesuré par tomographie fluorescente à l'aide d'un appareil TomoFluo3D développé par le CEA-LETI. La figure 16 montre un rendu 3D de la fluorescence au niveau de la tumeur dans le cube au dessus d'une image de fluorescence planaire de l'animal. La figure 17 représente la quantité de fluorescence déterminée en triplicat en pourcentage de la dose injecté (%DI, axe des ordonnées) dans la tumeur pour l'aptamère ACE8 et une séquence contrôle. Le symbole * correspond à un écart significatif du ratio entre ACE8 et la séquence contrôle mesuré par un test de student avec P<0.05.

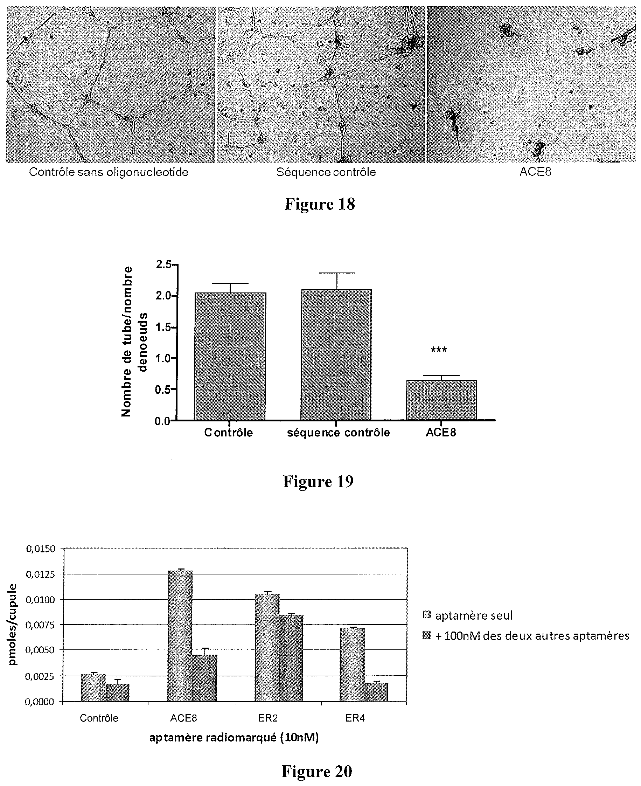

### Figures 18 et 19 : Utilisation de l'aptamère ACE8 comme inhibiteur de l'angiogénèse

Des cellules endothéliales humaines de la veine ombilicale (HUVEC) ont été cultivées, avec ou sans oligonucléotide (5µM), sur du Matrigel dans un mileu à faible teneur en hormones de croissance contenant 2% (v/v) de sérum de veau foetal et du facteur de croissance basique de fibroblaste (bFGF à 3 ng/mL). Dans ces conditions, les HUVEC forment un réseau de tubes endothéliaux qui peut être observé par microscopie et représente un modèle *in vitro* éprouvé de l'angiogénèse. La Figure 18 représente des photographies en microscopie des réseaux formés en l'absence d'oligonucléotide, en présence d'une séquence contrôle, et en présence de l'aptamère ACE8. La formation de ce réseau n'est pas affectée par la présence de la séquence contrôle. Par contre l'aptamère ACE8 inhibe fortement la formation de ce réseau. La figure 19 représente le nombre de tubes par noeud de cellules sur quatre champs de vue choisis aléatoirement, en l'absence d'oligonucléotide (contrôle), en présence d'une séquence contrôle, et en présence de l'aptamère ACE8. *** correspond à un écart significatif du ratio entre ACE8 et la séquence contrôle mesuré par un test de Student avec P<0.001.

### Figure 20 : Utilisation de l'aptamère ACE8 pour cribler des molécules pouvant se lier à l'annexine 2.

Chaque aptamère radiomarqué est incubé à une concentration de 10 nM sur les cellules CHO-ET_{B}R en présence (colonnes sombres) ou non (colonnes claires) des deux autres aptamères non-radiomarqués en excès (100 nM) pendant 30 minutes. Après plusieurs lavages, la radioactivité fixée aux cellules est comptée et la quantité d'aptamères fixés est déterminée (en pmoles/cupule). La figure 20 représente la liaison (axe des ordonnées, en pmoles/cupule) de l'aptamère ACE8, ER2, ou ER4, seul (première colonne) ou en présence des deux autres (deuxième colonne). Cette expérience démontre une compétition entre ACE8 et ER4 pour la liaison aux cellules suggérant que ER4 se lie à l'annexine 2.

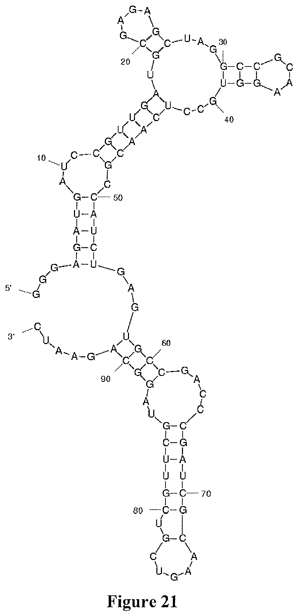

### Figure 21 : Prédiction de structure de l'aptamère ER4

La figure 21 représente la structure secondaire de l'aptamère ER4 prédite à l'aide du programme mfold (version 3.2, http://mfold.bioinfo.rpi.edu/cgi-bin/rna-form1.cgi).

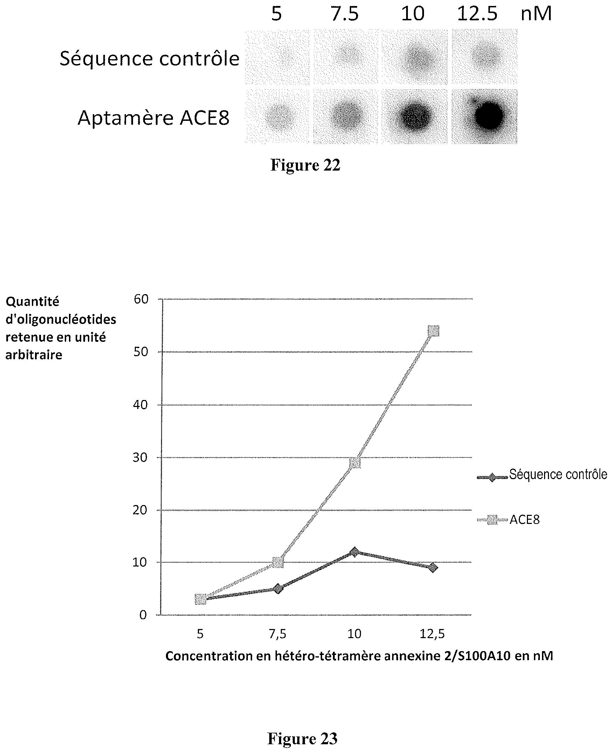

### Figures 22 et 23 : Mesure de l'affinité de l'aptamère ACE8 pour le complexe hétéro-tétramérique annexine 2/S100A10

L'aptamère ACE8 et une séquence contrôle ont été radiomarqués à l'extrémité 5' par un phosphore 32 ([³²P]). Ensuite, 1 pmole d'aptamère ACE8 ou de la séquence contrôle ont été pré-incubées avec différentes concentration d'un complexe hétéro-tétramérique annexine 2/S100A10. La fraction d'aptamère ACE8 ou de séquence contrôle fixée sur l'hétéro-tétramère annexine 2/S100A10 a été retenue sur un filtre de nitrocellulose et analysée et quantifiée par un *Phosphor Imager* STORM. La Figure 22 montre une captation plus importante de l'aptamère ACE8 sur l'hétéro-tétramère annexine 2/S100A10 par rapport à la séquence contrôle. La Figure 23 représente la quantification de la quantité d'oligonucléotides retenue sur le filtre en fonction de la concentration en hétéro-tétramère annexine 2/S100A10.

### EXEMPLES

### Exemple 1 : obtention de l'aptamère ACE8 spécifique de l'annexine 2

### 1. Répartition de l'aptamère ACE8 après 15 et 18 tours de sélection sur les cellules CHO-ETBR

Les inventeurs ont utilisé la technique de SELEX, notamment décrite dans la demande WO 2005/093097, dans le but de sélectionner des aptamères contre le récepteur à l'endothéline de type B (ET_{B}R). Pour cela, des cellules CHO surexprimant de manière stable l'ET_{B}R ont été utilisées pour la sélection et des cellules CHO n'exprimant pas l'ET_{B}R pour la contre-sélection (**Figure 1**).

Au cours de ce SELEX, les inventeurs ont fait varier différents paramètres dans le but d'augmenter progressivement la pression de sélection (**Tableau 2**). Par rapport au protocole décrit dans la demande WO 2005/093097, les inventeurs ont apporté quelques modifications : durant quelques tours, l'étape de sélection et de contre-sélection ont été inversées (tours 14 et 15) et les aptamères ont été décrochés des cellules par l'ajout d'endotheline-1 pour les trois derniers tours (16 à 18). Les conditions de sélection sont présentées dans le **Tableau 1** ci-dessous :

**Tableau 1 : évolution de la pression de sélection au cours du SELEX**

| N° du cycle | nombre de cellules pour la sélection (millions) | nombre de cellules pour la contre-selection (millions) | inversion selection-contre-selection | volume d'incubation (ml) | concentration en ARN-2'F-Py (µM) | durée d'incubation (minutes) | nombre de lavage | condition particulière de lavage | compétiteur ajouté durant l'incubation (100µg/ml de tRNA) | mode d'elution |
|---|---|---|---|---|---|---|---|---|---|---|
| 1 | 10 | 10 | | 3 | 1 | 30 | 2 | | | tri-reagent |
| 2 | 10 | 10 | | 3 | 1 | 30 | 3 | | | tri-reagent |
| 3 | 10 | 10 | | 3 | 1 | 25 | 3 | | | tri-reagent |
| | 5 | 5 | | 3 | 1 | 25 | 3 | | | tri-reagent |
| | 5 | 5 | | 3 | 1 | 25 | 4 | | | tri-reagent |
| | 5 | 5 | | 3 | 1 | 20 | 4 | | | tri-reagent |
| | 5 | 5 | | 3 | 0,5 | 20 | 5 | | | tri-reagent |
| | 8 5 | 5 | | 3 | 0,5 | 15 | 5 | | | tri-reagent |
| | 5 | 5 | | 3 | 0,5 | 15 | 5 | 5 min pour le dernier lavage | | tri-reagent |
| 10 | 5 | 5 | | 3 | 0,5 | 10 | 5 | 5 min pour le dernier lavage | | tri-reagent |
| 11 | 5 | 5 | | 3 | 0,5 | 10 | 5 | 5 min pour le dernier lavage | + | tri-reagent |
| 12 | 5 | 5 | | 3 | 0,5 | 10 | 5 | 5 min pour le dernier lavage | + | tri-reagent |
| 13 | 5 | 5 | | 3 | 0,5 | 10 | 5 | 5 min pour les 2 derniers lavages | + | tri-reagent |
| 14 | 5 | 5 | + | 3 | 0,5 | 10 | 5 | 5 min pour les 2 derniers lavages | + | tri-reagent |
| 15 | 5 | 5 | + | 3 | 0,5 | 10 | 5 | 5 min pour les 2 derniers lavages | + | tri-reagent |
| 16 | 5 | 5 | | 3 | 0,025 | 10 | 5 | 5 min pour les 2 derniers lavages | + | elution par 250nM d'endotheline-1 |
| 17 | 5 | 5 | | 3 | 0,025 | 10 | 5 | 5 min pour les 2 derniers lavages | + | elution par 250nM d'endotheline-1 |
| 18 | 5 | 5 | | 3 | 0,025 | 10 | 5 | 5 min pour les 2 derniers lavages | + | elution par 250nM d'endotheline-1 |

Les banques issues du tour 15 et du tour 18 ont été clonées et séquencées. De manière intéressante une séquence (ACE8) est deux fois plus abondante dans le tour 18 que dans le tour 15.

### 2. Effet de l'endothéline sur la fixation des aptamères

Les inventeurs ont testé l'affinité d'une trentaine de séquences pour ces cellules à 25nM. Ceci leur a permis d'identifier une dizaine d'aptamères particulièrement intéressant avec une forte affinité pour les cellules CHO-ET_{B}R. Parmi tous ces aptamères, ceux dont l'affinité pour les cellules pouvaient être liée avec l'expression du récepteur à l'endothéline de type B ont été recherchés. Pour cela, les inventeurs ont comparé l'affinité des aptamères pour des cellules CHO-ET_{B}R préalablement incubées en présence d'endothéline. Lorsque l'endothéline se fixe sur le récepteur ET_{B}R, le récepteur est très rapidement internalisé (**Figure 3**). Suite à l'internalisation du récepteur à l'endothéline, trois aptamères ont montré une perte d'affinité pour les cellules (ACE8 (SEQ ID NO : 3), ER2 (SEQ ID NO : 4) et ER4 (SEQ ID NO : 8)) (**Figure 2**). Cependant, tous ces aptamères ont également de l'affinité pour les cellules CHO-WT, qui n'expriment pas le récepteur. De plus, suite au traitement à l'endothéline des CHO-WT, l'affinité de ces aptamères ne diminue pas. Toutes ces expériences semblent démontrer que la cible de ces aptamère n'est pas ET_{B}R mais une autre protéine qui pourrait être endocytée en même temps qu'ET_{B}R après fixation de l'endothéline.

Afin d'évaluer l'intérêt potentiel de ces aptamères, les inventeurs ont mesuré leur affinité sur des cellules MCF-7. Ces cellules humaines issues de lignée de cancer du sein sont connues pour exprimer les récepteurs à l'endothéline de type A et B. De façon intéressante l'aptamère ACE8 et l'aptamère ER4 ont montré une forte affinité pour les cellules MCF-7. Ceci démontre que ces aptamères peuvent se fixer sur une cible d'origine humaine et pas seulement à la surface de cellule de hamster. De plus, des expériences de binding ont révélé que la cible de l'aptamère ACE8 était 10 fois plus abondante à la surface des cellules MCF-7 qu'à la surface des cellules CHO. Suite à ce résultat, les inventeurs se sont plus particulièrement intéressés à l'aptamère ACE8.

### 3. Séquence de l'aptamère ACE8

La séquence de l'aptamère ACE8 est la suivante :

Il est à noter qu'afin d'améliorer la résistance de l'aptamère ACE8 aux nucléases, les riboses des pyrimidines portent un atome de fluor sur le carbone en position 2' (les riboses des purines sont quant à eux porteurs, comme c'est le cas dans l'ARN naturel, d'une fonction hydroxyle (OH) sur le carbone en position 2').

La **Figure 4** représente une prédiction de structure secondaire de l'aptamère ACE8 réalisée par le logiciel mfold (version 3.4) (Nucleic Acids Res. (2003) 31:3406-15) et vsfold5 RNA Pseudoknot Prédiction (PLoS One (2007), 2:905).

### 4. Caractérisation de l'aptamère ACE8

### a- Validation de l'utilisation de l'aptamère ACE8 pour établir un profil d'expression à la surface de différentes lignées cellulaires.

L'affinité et le Cmax de l'aptamère ACE8 ont été déterminés pour différentes lignées cancéreuses (**Tableau 2**).

**Tableau. 2 : Affinité et nombre de cibles par cellule de l'aptamère ACE8 pour différentes lignées cellulaires**

| espèces | Nom de la lignée | Kd (nM) | Cmax (pM) |
|---|---|---|---|
| humaine | MCF-7 | 4,9 +/- 1,7 | 484 +/- 81 |
| | A-431 | 5,3 +/- 1,1 | 450 +/- 35 |
| | MDA-MB-231 | 4,4 +/- 0,6 | 167 +/- 16 |
| | U87-MG | 12,7 +/- 4,4 | 245 +/- 55 |
| | HEK-293 | 4,4 +/- 0,8 | 269 +/- 21 |
| souris | 4T1 | 3,7 +/- 0,3 | 247 +/- 11 |
| | EMT6 | 7,5 +/- 1,3 | 261 +/- 53 |
| Hamster | CHO-ETBR | 3,4 +/- 0,8 | 86 +/- 13 |
| Rat | PC12-MEN-2A | 18,8 +/- 2,6 | 914 +/- 134 |

| | | | |
|---|---|---|---|
| Pour chaque type cellulaire, les courbes de binding ont été obtenues. A partir de la liaison spécifique, et à l'aide de la représentation de Scatchard, le Kd et le nombre de cibles par cellule ont été déterminés pour chacun des types cellulaires. La cible de l'aptamère ACE8 semble très abondante à la surface de deux lignées humaines de cancer (MCF-7 et A-431). | | | |

L'aptamère ACE8 a une affinité d'environ 4 nM pour la plupart des lignées cellulaires, excepté pour les cellules PC12 MEN 2A et les cellules U87 où le Kd dépasse la dizaine de nM. Le nombre de cibles est très différent selon le type cellulaire allant d'un Cmax de 86+/- 13 pM pour les CHO à 484 +/- 81 pM pour les MCF-7. De façon intéressante, la cible de l'aptamère ACE8 semble très abondante à la surface de la lignée humaine de cancer du sein MCF-7 et sur la lignée humaine de carcinome de l'épiderme A-431.

### b- Validation de l'utilisation de l'aptamère ACE8 en microscopie

Une fois marqué par un composé fluorescent (phycoérythrine), l'aptamère a pu être utilisé en microscopie (**Figure 5**). Un contraste a pu être observé avec les cellules A-431 et MCF-7, les types cellulaires qui ont le plus de cibles (450 +/- 35 et 484 +/- 81 pM, respectivement). Etant donné le nombre très important de cibles à la surface des MCF-7 et le bon contraste observé en microscopie, les inventeurs ont choisi d'approfondir l'étude de l'aptamère ACE8 sur ce type cellulaire, qui représente en plus l'avantage d'être une lignée de cancer du sein, indiquant ainsi que l'aptamère ACE8 pourrait éventuellement cibler une protéine impliquée dans ce cancer.

### 5. Identification de la cible de l'aptamère ACE8

### a. Validation du protocole : Fixation de l'aptamère ACE8 sur cellules en suspension et détermination de la nature de la cible

Avant d'essayer de purifier la cible de l'aptamère ACE8, les inventeurs ont tout d'abord validé, d'une part, que l'aptamère ACE8 se fixe toujours lorsque les cellules sont en suspension (**Figures 6** **et** **7**). D'autre part, lorsque les cellules sont incubées avec de la trypsine et de la protéinase K, la fixation de l'aptamère est perdue ce qui démontre que la cible de l'aptamère est une protéine.

### b. Protocole de purification de la cible de l'aptamère

Afin d'identifier la cible de l'aptamère ACE8, un protocole dérivé de Berezovski et al. (2008) J. Am. Chem. Soc., *Aptamer-Facilitated Biomarker Discovery* (AptaBiD) a été utilisé, dans lequel de la BSA a été rajoutée comme compétiteur pour éviter la fixation non spécifique de protéines sur les billes. Le protocole est résumé dans la **Figure 8**.

### c. Gel SDS-PAGE et spectrométrie de masse

Les protéines éluées à la fin du protocole résumé dans la **Figure 8** ont été analysées par électrophorèse (**Figure 9**). 6 bandes spécifiques de l'aptamère ACE8 ont été identifiées. Ces bandes ont été découpées et analysées par spectrométrie de masse. Parmi les différentes protéines identifiées, 4 sont impliquées dans les RAFTs lipidiques. Ceci suggère que la cible de l'aptamère ACE8 est une protéine localisée dans les RAFTs. Parmi ces protéines, les inventeurs ont choisi de s'intéresser plus particulièrement à l'annexine 2 au vue de l'intensité de la bande pour cette protéine.

### d. Validation de la cible de l'aptamère ACE8

Afin de valider la cible de l'aptamère ACE8, des siRNA (SEQ ID NO : 9 et 10) ciblant l'annexine 2 ont été utilisés. Comme le montre la **Figure 10**, une diminution de 60% de l'expression de l'annexin 2 est observée 72 h après transfection de ces siRNA. De façon remarquable, lorsque l'aptamère ACE8 est incubé avec des cellules transfectées avec le siRNA ciblant l'annexine 2, la fixation de l'aptamère est réduite de 40% par rapport à des cellules MCF-7 transfectées par un siRNA contrôle (**Figure 11**). Ces résultats indiquent que la cible de l'aptamère ACE8 est bien l'annexine 2.

### Exemple 2 : utilisation de l'aptamère ACE8 en microscopie

En utilisant différentes techniques de marquage de l'aptamère ACE8, directe ou non (phycoérythrine, alexafluor, quantum Dot) les inventeurs ont également montré que l'aptamère ACE8 peut être utilisé de façon très simple en microscopie sur cellules vivantes (**Figure 12**).

### Exemple 3 : utilisation de l'aptamère ACE8 comme molécule d'adressage de nanoparticules et internalisation de composés

L'aptamère ACE8 a conservé ses propriétés de ciblage une fois greffé à la surface d'un Quantum Dot, cela démontre qu'il pourrait être utilisé pour adresser de façon spécifique d'autres nanoparticules utiles pour l'imagerie ou la thérapie. L'aptamère ACE8 étant endocyté par les cellules au fil du temps, il est également capable de faire rentrer ces nanoparticules ou d'autres composés, tels que des composés pharmacologiques ou des agents de contraste dans les cellules (**Figure 13**).

### Exemple 4 : utilisation de l'aptamère ACE8 en cytométrie en flux

L'aptamère ACE8 marqué de manière fluorescente a été utilisé en cytométrie en flux (CMF) après incubation sur les cellules MCF-7 et sur les cellules A-431 à une concentration de 5 nM (**Figure 14**). On peut voir une nette augmentation de l'intensité de fluorescence des cellules lorsqu'elles sont incubées avec l'aptamère ACE8 par rapport aux cellules incubées avec la séquence contrôle (SEQ ID NO : 11). De plus, cette augmentation d'intensité est plus ou moins importante selon la quantité de cible à la surface des cellules. Ces résultats sont en accord avec les données de liaison : l'intensité de fluorescence des cellules MCF-7 est supérieure à celle des cellules A-431 après incubation avec l'aptamère ACE8, et les MCF-7 ont un nombre de cibles plus importantes que les cellules A-431.

### Exemple 5 : utilisation de l'aptamère ACE8 en imagerie in vivo

L'aptamère ACE8 a été utilisé pour l'imagerie *in vivo* de tumeur. L'aptamère ACE8 marqué de manière fluorescente a été injecté par voie intraveineuse dans des souris *nudes* développant des tumeurs issues de xénogreffes sous-cutanée de cellules MCF-7. La biodistribution de l'aptamère a été mesurée par imagerie fluorescente. Par imagerie semi-quantitative *in vivo* (**Figure 15**), les inventeurs ont pu observer que l'aptamère diffuse très vite du compartiment sanguin et est très vite éliminé par voie urinaire et partiellement par voie hépatobiliaire. De manière intéressante, la comparaison du signal dans la tumeur par rapport à une zone de référence au niveau de la tête de l'animal (rapport tumeur/référence interne) augmente plus rapidement pour l'aptamère ACE8 que pour une séquence contrôle (SEQ ID NO : 11) et une différence significative est observée 15 minutes après l'injection (1,61 +/- 0,15 par rapport à 1,18 +/- 0,04, respectivement, voir le **Tableau 3**). Après 15 minutes, ce rapport augmente progressivement mais de manière similaire pour l'aptamère ACE8 et la séquence contrôle (**Figure 15** **et Tableau 1**).

**Tableau 3 : rapport signal dans la tumeur/référence interne au cours du temps**

| **Temps (min)** | **Ratio tumeur/reference interne Séquence contrôle** | **Ratio tumeur/reference interne ACE8** | **Test statistique de student (P<0.05)** |
|---|---|---|---|
| 5 | 1,10+/-0,12 | 1,34 +/- 0,14 | Ns |
| 10 | 1,17+/-0,10 | 1,48 +/- 0,16 | Ns |
| 15 | 1,18 +/- 0,04 | 1,61 +/- 0,15 | * |
| 30 | 1,32 +/- 0,08 | 1,73 +/- 0,22 | * |
| 60 | 1,42 +/- 0,09 | 1,79 +/- 0,21 | * |
| 90 | 1,43 +/- 0,18 | 1,85 +/- 0,15 | * |
| 120 | 1,52 +/- 0,11 | 1,90 +/- 0,17 | * |
| 150 | 1,54 +/- 0,15 | 1,96 +/- 0,28 | * |
| 180 | 1,57 +/- 0,17 | 1,97 +/- 0,29 | * |

| | | | |
|---|---|---|---|
| Le Signal de fluorescence mesuré dans la tumeur a été divisé par le signal fluorescent mesuré dans une zone au niveau de la tête de l'animal (voir **Figure 15**). Ce ratio a été mesuré à différents temps en triplicat. * correspond à un écart significatif du ratio entre ACE8 et la séquence contrôle mesuré par un test de student avec P<0.05. Ns : non significatif. | | | |

Ce résultat démontre que l'aptamère ACE8 doit rapidement se fixer à sa cible dans la tumeur.

Afin de mieux quantifier la biodistribution de l'aptamère dans la tumeur, des mesures ont été effectuées par tomographie de fluorescence au temps 3h post-injection (**Figures 16 et 17**). Nous avons ainsi pu mesurer qu'environ 0,67% ± 0,27 de la dose injectée d'aptamère est présente dans la tumeur 3h post-injection à comparer à 0,07% ± 0,11 pour la séquence contrôle.

### Exemple 6 : utilisation de l'aptamère ACE8 comme inhibiteur de l'angiogénèse

L'utilisation de l'aptamère ACE8 comme inhibiteur de l'angiogénèse a été validée sur un modèle *in vitro* de formation de tubes endothéliaux. Des cellules endothéliales humaines de la veine ombilicale (HUVEC) ont été cultivées, avec ou sans oligonucléotide (5µM), sur du Matrigel™ (Becton-Dickinson) dans un milieu à faible teneur en hormone de croissance comportant 2% (v/v) de sérum de veau foetal et du facteur de croissance basique de fibroblaste (bFGF à 3 ng/mL). Dans ces conditions, les HUVEC forment un réseau de tubes endothéliaux qui peut être observé par microscopie et représente un modèle *in vitro* éprouvé de l'angiogénèse. La formation de ce réseau n'est pas affectée par la présence de la séquence contrôle. Par contre l'aptamère ACE8 inhibe fortement la formation de ce réseau (**Figure 18 et 19**).

### Exemple 7 : Utilisation de l'aptamère ACE8 pour cribler des molécules pouvant se lier à l'annexine 2.

Il a été montré qu'il est possible d'utiliser l'aptamère ACE8 pour cribler des molécules pouvant se lier à l'annexin 2 par des expériences de compétition. Pour ceci, les inventeurs ont étudié si une telle compétition avait lieu entre les aptamères entre eux.

Ainsi, lorsque l'aptamère ER2 radiomarqué est incubé en présence des aptamères ACE8 et ER4, l'affinité est la même que l'aptamère seul, ce qui signifie que l'aptamère ER2 n'est pas en compétition avec les aptamères ACE8 et ER4. Par contre, lorsque l'aptamère ACE8 est incubé en présence des deux autres, une baisse d'affinité est observée. Il en est de même pour l'aptamère ER4 (**Figure 20**).

Les résultats de ces expériences montrent une compétition entre les aptamères ACE8 et ER4, suggérant que l'aptamère ER4 aurait également pour cible l'annexine 2

L'aptamère ER4 et représenté dans la **Figure 21** et est constitué de la séquence :

### Exemple 8 : Mesure de l'affinité de l'aptamère ACE8 pour le complexe hétéro-tétramérique annexine 2/S100A10

Les inventeurs ont également montré une fixation spécifique et directe d'ACE8 sur le complexe hétéro-tétramérique annexine 2/S100A10 (**Figures 22 et 23**).

Pour ceci, les oligonucléotides (aptamère ACE8 ou séquence contrôle (SEQ ID NO : 11)) ont été déphosphorylés à leur extrémité 5' pendant 30 min à 37°C par 2,5 U d'*Antartic Phosphatase* (New-England Biolabs) par nmole d'ARN 2'F-Py. Après inactivation de l'enzyme à 88°C pendant 15 min, l'ARN 2'F-Py a été précipité à l'éthanol en présence de 5µl/ml d'un co-précipitant neutre (*Linear Polyacrylamide* ou LPA) (Ambion). Les oligonucléotides ont ensuite été marqués par de l'ATP [gamma-³²P] par la T4 kinase (Invitrogen) selon les instructions du fournisseur. Les ARN 2'F-Py (2,3 MBq soit 63nCi/pmole ou 63nCi/pmole-1) ont été par la suite purifiés par chromatographie d'exclusion (*Bio-Spin Sp30 Chromatography Columns* (Biorad)). 1pmole d'aptamère ACE8 ou de la séquence contrôle ont alors été pré-incubées pendant 30 min à température ambiante avec différentes concentrations (0, 5, 7.5, 10 et 12.5 nM) d'un complexe hétéro-tétramérique annexin 2/S100A10 (2 protéines d'annexine 2 avec deux protéines de S100A10) purifié à partir de poumon de vache (Interchim, France, ref: A80109B). La pré-incubation a été réalisée dans 200µl de milieu RPMI 1640 (Invitrogen) en présence de 3µg d'ARN de transfert de levure (Sigma-Aldrich) ce qui représente une quantité 100 fois plus élevée d'ARN par rapport à l'aptamère ou à la séquence contrôle. Le mélange a ensuite été filtré sur des membranes de nitrocellulose *HAWP* 0,45µ (Millipore) et la membrane a été lavée par filtration de 5 volumes de 200µl de RPMI 1640. La membrane de nitrocellulose permet de retenir les protéines et de laisser passer les ARN. Ainsi, seule la fraction d'aptamère ACE8 ou de séquence contrôle fixée sur l'hétéro-tétramère annexine 2/S100A10 est retenue sur le filtre de nitrocellulose. Ce filtre est ensuite séché et la fraction d'aptamère ACE 8 ou de séquence contrôle fixée sur l'hétéro-tétramère annexine 2/S100A10 peut être quantifiée par un *Phosphor Imager* STORM (GE Healthcare).

Les résultats (**Figures 22 et 23**) démontrent que l'aptamère ACE8 interagit avec l'hétéro-tétramère annexine 2/S100A10 avec une très forte affinité par rapport à une séquence contrôle.

## Revendications

1. Aptamère comprenant un acide nucléique comprenant, ou constitué de :
- la séquence GGAACGCAAGAACUGAGGCCAUGAGGCGCCUUCCCUUGCUCA GGACGC (SEQ ID NO : 1), ou
- la séquence AGCUAGGCCGCAAGGUGCCUCAACGCCAUCUGAGUGCCGACC CGAUCGC (SEQ ID NO : 2), ou
- une séquence comprenant, ou constituée d'au moins 25 nucléotides consécutifs d'une séquence ayant au moins 80% d'identité avec SEQ ID NO : 1 ou SEQ ID NO : 2, sous réserve qu'un acide nucléique constitué de cette séquence se lie à l'annexine 2.

2. Aptamère selon la revendication 1, comprenant un acide nucléique constitué de :
- la séquence GGGAGAUGAUCCGUUGAUGCGAGGGAACGCAAGAACUGAGGC CAUGAGGCGCCUUCCCUUGCUCAGGACGCAAGUCGUCGUUCGUAGGCAG AAUC (SEQ ID NO : 3), ou
- la séquence GGGAGAUGAUCCGUUGAUGCGAGAGCUAGGCCGCAAGGUGCC UCAACGCCAUCUGAGUGCCGACCCGAUCGCAAGUCGUCGUUCGUAGGCA GAAUC (SEQ ID NO : 4).

3. Aptamère selon la revendication 1 ou 2, dans lequel l'acide nucléique est lié à au moins un groupement additionnel.

4. Aptamère selon l'une des revendications 1 à 3, dans lequel le groupement additionnel est sélectionné dans le groupe constitué d'un marqueur détectable, d'un composé pharmacologique, et d'un composé susceptible de modifier les caractéristiques pharmacocinétiques d'un acide nucléique auquel il est lié.

5. Aptamère selon l'une des revendications 1 à 4, constitué de l'acide nucléique et d'au moins un groupement additionnel.

6. Aptamère selon l'une des revendications 1 à 5, pour son utilisation à titre de médicament ou d'agent diagnostique.

7. Composition pharmaceutique ou diagnostique comprenant, à titre de substance active, au moins un aptamère tel que défini dans l'une des revendications 1 à 5 en association avec au moins un véhicule pharmaceutiquement acceptable.

8. Utilisation *in vitro* d'un aptamère tel que défini dans l'une des revendications 1 à 5, pour détecter la présence d'annexine 2 ou déterminer la quantité d'annexine 2 dans un échantillon biologique.

9. Méthode *in vitro* de détection ou de quantification d'annexine 2 dans un échantillon biologique, comprenant :
- mettre en contact l'échantillon biologique avec un aptamère tel que défini dans l'une des revendications 1 à 5 ;
- quantifier, ou détecter la présence ou l'absence d'aptamère fixé dans l'échantillon ;
- en déduire la quantité, ou la présence ou l'absence d'annexine 2 dans l'échantillon.

10. Méthode selon la revendication 9, dans laquelle la détection d'aptamère fixé dans l'échantillon est effectuée par mise en oeuvre d'une PCR destinée à amplifier l'aptamère.

11. Utilisation d'un aptamère tel que défini dans l'une des revendications 1 à 5, pour le criblage de ligands de l'annexine 2.

12. Méthode de criblage de ligands de l'annexine 2 comprenant :
- la mise en présence d'annexine 2 et, concomitamment ou successivement, d'un ligand à cribler et d'un aptamère tel que défini dans l'une des revendications 1 à 5 ;
- déterminer la quantité d'aptamère fixé à l'annexine 2 ;
- en déduire si le ligand est un ligand de l'annexine 2.

## Patentansprüche

1. Aptamer, umfassend eine Nukleinsäure umfassend oder bestehend aus:
- der Sequenz GGAACGCAAGAACUGAGGCCAUGAGGCGCCUUCCCUUGCUCA GGACGC (SEQ ID NO: 1), oder
- der Sequenz AGCUAGGCCGCAAGGUGCCUCAACGCCAUCUGAGUGCCGACC CGAUCGC (SEQ ID NO: 2), oder
- einer Sequenz umfassend oder bestehend aus wenigstens 25 aufeinander folgenden Nukleotiden einer Sequenz, welche wenigstens 80 % Identität mit SEQ ID NO: 1 oder SEQ ID NO: 2 aufweist, mit der Maßgabe, dass eine Nukleinsäure, welche aus dieser Sequenz besteht, an Annexin 2 bindet.

2. Aptamer gemäß Anspruch 1, umfassend eine Nukleinsäure bestehend aus:
- der Sequenz GGGAGAUGAUCCGUUGAUGCGAGGGAACGCAAGAACUGAGGC CAUGAGGCGCCUUCCCUUGCUCAGGACGCAAGUCGUCGUUCGUAGGCAG AAUC (SEQ ID NO: 3), oder
- der Sequenz GGGAGAUGAUCCGUUGAUGCGAGAGCUAGGCCGCAAGGUGCC UCAACGCCAUCUGAGUGCCGACCCGAUCGCAAGUCGUCGUUCGUAGGCA GAAUC (SEQ ID NO: 4).

3. Aptamer gemäß Anspruch 1 oder 2, in welchem die Nukleinsäure an wenigstens eine weitere Gruppe gebunden ist.

4. Aptamer gemäß einem der Ansprüche 1 bis 3, in welchem die weitere Gruppe ausgewählt ist aus der Gruppe bestehend aus einem detektierbaren Marker, einer pharmakologischen Verbindung und einer Verbindung, welche geeignet ist, die pharmakokinetischen Merkmale einer Nukleinsäure zu modifizieren, an welche sie gebunden ist.

5. Aptamer gemäß einem der Ansprüche 1 bis 4, bestehend aus der Nukleinsäure und wenigstens einer weiteren Gruppe.

6. Aptamer gemäß einem der Ansprüche 1 bis 5, zur Verwendung als Medikament oder diagnostisches Mittel.

7. Pharmazeutische oder diagnostische Zusammensetzung, umfassend als Wirkstoff wenigstens ein Aptamer wie in einem der Ansprüche 1 bis 5 definiert, assoziiert mit wenigstens einem pharmazeutisch akzeptablen Träger.

8. *In vitro* Verwendung eines Aptamers wie in einem der Ansprüche 1 bis 5 definiert, zur Detektion der Anwesenheit von Annexin 2 oder zur Bestimmung der Menge von Annexin 2 in einer biologischen Probe.

9. *In vitro* Verfahren zur Detektion oder zur Quantifizierung von Annexin 2 in einer biologischen Probe, umfassend:
- Inkontaktbringen der biologischen Probe mit einem Aptamer wie in einem der Ansprüche 1 bis 5 definiert,
- Quantifizieren oder Detektieren der Anwesenheit oder Abwesenheit des gebundenen Aptamers in der Probe,
- anhand dessen Bestimmen der Menge oder der Anwesenheit oder der Abwesenheit von Annexin 2 in der Probe.

10. Verfahren gemäß Anspruch 9, in welchem die Detektion des gebundenen Aptamers in der Probe ausgeführt wird, indem eine PCR zur Amplifikation des Aptamers durchgeführt wird.

11. Verwendung eines Aptamers wie in einem der Ansprüche 1 bis 5 definiert, zur Musterung von Liganden von Annexin 2.

12. Verfahren zur Musterung von Liganden von Annexin 2, umfassend:
- Zusammenbringen von Annexin 2 und, gleichzeitig oder nacheinander, einem Liganden, der gemustert werden soll, und einem Aptamer wie in einem der Ansprüche 1 bis 5 definiert,
- Bestimmen der Menge des Aptamers, welches an Annexin 2 gebunden ist,
- anhand dessen Bestimmen, ob der Ligand ein Ligand von Annexin 2 ist.

## Claims

1. An aptamer comprising a nucleic acid, comprising or consisting of:
- the sequence GGAACGCAAGAACUGAGGCCAUGAGGCGCCUUCCCUUGCUCAGGACGC (SEQ NO: 1), or
- the sequence AGCUAGGCCGCAAGGUGCCUCAACGCCAUCUGAGUGCCGACCCGAUCGC (SEQ ID NO: 2), or
- a sequence comprising, or consisting of, at least 25 consecutive nucleotides of a sequence having at least 80% identity with SEQ NO: 1 or SEQ NO: 2, provided that a nucleic acid consisting of this sequence binds to annexin 2.

2. The aptamer according to claim 1, comprising a nucleic acid consisting of the sequence or
- the sequence

3. The aptamer according to claim 1 or 2, wherein the nucleic acid is linked to at least one additional group.

4. The aptamer according to one of the claims 1 to 3, wherein the additional group is selected from the group consisting of a detectable label, a pharmacological compound, and a compound capable of modifying the pharmacokinetic characteristics of a nucleic acid to which it is linked.

5. The aptamer according to one of the claims 1 to 4, consisting of the nucleic acid and of at least one additional group.

6. The aptamer according to one of the claims 1 to 5, for its use as a medicament or a diagnostic agent.

7. Pharmaceutical or diagnostic composition comprising, as an active substance, at least one aptamer as defined in one of the claims 1 to 5 in combination with at least one pharmaceutically acceptable vehicle.

8. *In vitro* use of an aptamer as defined in one of the claims 1 to 5, for detecting the presence of annexin 2 or determining the amount of annexin 2 in a biological sample.

9. *In vitro* method of detecting or quantifying annexin 2 in a biological sample, comprising:
- bringing the biological sample into contact with an aptamer as defined in one of the claims 1 to 5;
- quantifying, or detecting the presence or absence of aptamer bound in the sample;
- deducing therefrom the amount, or the presence or absence of annexin 2 in the sample.

10. The method according to claim 9, wherein detecting aptamer bound in the sample is carried out by preforming a PCR intended to amplify the aptamer.

11. Use of an aptamer as defined in one of the claims 1 to 5, for screening ligands of annexin 2.

12. Method of screening ligands of annexin 2 comprising:
- bringing together annexin 2 and, concurrently or successively, a ligand to be screened and an aptamer as defined in one of the claims 1 to 5;
- determining the amount of aptamer bound to annexin 2;
- deducing there from whether the ligand is a ligand of annexin 2.
